# EUROPÄISCHE PATENTSCHRIFT

(11) **EP 2 139 505 B1**
(45) Veröffentlichungstag und Bekanntmachung des Hinweises auf die Patenterteilung: **25.06.2014**
(21) Anmeldenummer: 08716588.2
(22) Anmeldetag: 18.03.2008
(51) Int. Cl.: A61K 36/68, A61K 36/482, A61P 1/10

(54) **NEUE PHARMAZEUTISCHE ZUSAMMENSETZUNG ZUR VERWENDUNG ALS LAXATIVUM**
NOVEL PHARMACEUTICAL COMPOSITION FOR USE AS A LAXATIVE
NOUVELLE COMPOSITION PHARMACEUTIQUE POUR UNE UTILISATION COMME LAXATIF

(30) Priorität: 02.05.2007 DE 102007020842; 18.05.2007 DE 102007023397
(43) Veröffentlichungstag der Anmeldung: 06.01.2010
(73) Patentinhaber: Madaus GmbH, 51067 Köln (DE)
(72) Erfinder: MORICK, Wolfgang, 79219 Staufen (DE); PRENNER, Lars-Norbert, 53225 Bonn (DE); HUBBERT, Michael, 51503 Rösrath-Kleineichen (DE); GEBHART, Kurt N., 50933 Köln (DE); BEHM, Wolf, 53913 Swisttal (DE)
(74) Vertreter: Von Rohr Patentanwälte Partnerschaft mbB
(86) Internationale Anmeldenummer: PCT/EP2008/002126
(87) Internationale Veröffentlichungsnummer: WO 2008/135115

(56) Entgegenhaltungen:
- EP-A- 0 387 933
- WO-A-2007/013093
- WO-A-2008/080809
- FR-A- 2 646 352
- US-A- 4 595 592
- US-A- 5 232 699
- US-A- 5 320 847

## Beschreibung

Die vorliegende Erfindung betrifft eine neue Zusammensetzung, insbesondere pharmazeutische Zusammensetzung, zur Verwendung als Laxativum (synonym auch als "Abführmittel" oder "Laxans" bezeichnet) und ein Verfahren zu ihrer Herstellung sowie die Verwendung dieser Zusammensetzung als Laxativum (d. h. Abführmittel bzw. Laxans).

Laxativa - synonym auch als "Laxantien" oder "Abführmittel" bezeichnet - gibt es in den vielfältigsten Erscheinungsformen, hierunter auch zahlreiche pflanzliche Abführmittel.

So ist beispielsweise die Verwendung von Plantagosamen (Plantaginis ovatae Semen bzw. Semen plantaginis ovatae, synonym auch als Indischer Flohsamen, Indisches Psyllium, Blondes Psyllium, Ispaghula oder Semen Ispaghulae bezeichnet) und/oder seiner Samenschalen (Plantagosamenschalen, Plantaginis ovatae seminis integumentum) für Arzneimittel zur Regulierung der Darmtätigkeit bekannt. Plantagosamen besitzt eine beträchtliche Quellfähigkeit und übt einen physikalischen Dehnungsreiz auf die empfindlichen Rezeptoren der Darmwände aus. Nach einem bekannten Verfahren (vgl. DE-PS 11 03 520) werden die Samen fein vermahlen, mit Wasser zu einem viskosen Brei angeteigt und in Strangform getrocknet, zerkleinert und schließlich dragiert.

Die Wirkung der Sennespflanze (Sennapflanze, Cassia senna L. und Cassia angustifolia Vahl), insbesondere ihrer Früchte (Fructus Sennae, synonym auch als Sennesschoten oder Sennesbälge bezeichnet) (z. B. Alexandriner-Sennesfrüchte = Sennae fructus acutifoliae und/oder Tinnevelly-Sennesfrüchte = Sennae fructus angustifoliae) bzw. ihrer Fruchthülsen wie auch ihrer Fiederblätter (Folia Sennae), als pflanzliches Abführmittel ist ebenfalls bekannt.

Darüber hinaus kennt man auch Laxativa, welche die beiden vorgenannten Wirkungsprinzipien vereinen, beispielsweise Laxativa, bei denen die physikalische Wirkung der Plantagosamen durch das pharmakologisch anregende Wirkprinzip der Sennoside, der Inhaltsstoffe der Sennesfrüchte, unterstützt wird, um eine bessere Gesamtwirkung zu erreichen.

So ist es bereits bekannt, Mischungen aus vermahlenem Plantagosamen und Sennesfrüchten als einfaches Gemisch, bei dem die Bestandteile nebeneinander vorliegen, zu konfektionieren. Jedoch ist bei derartigen Gemischen die Rieselfähigkeit und Suspendierbarkeit in Wasser nicht optimal einzustellen, was für die orale Applikation aber wünschenswert wäre. Auch ist die Verarbeitung dieser Gemische zu applikationsfertigen Pulvern nicht oder nicht ohne weiteres möglich.

Um den vorgenannten Nachteilen abzuhelfen, ist in der DE 30 01 357 C2 ein Laxativum in Form eines Abführmittelgranulates auf der Basis von Sennesfrüchten, Plantagosamen und gegebenenfalls Plantagosamenschalen mit erhöhter Retardwirkung vorgeschlagen worden, bei denen die Sennapartikel von Plantagosamen, insbesondere Schleimstoffen der Plantagosamen, umschlossen bzw. umhüllt vorliegen. Die dort beschriebene Zusammensetzung ist ein effizientes Laxativum. Da die Zusammensetzung aber als Granulat mit etwa 1 mm bis etwa 3 mm Korngröße der Granulatteilchen, d. h. als asymmetrische Aggregate von Pulverpartikeln, vorliegt, muß sie mit reichlich Wasser eingenommen werden. Ansonsten besteht die Gefahr, daß es bei unsachgemäßer oraler Einnahme bzw. Applikation, also bei der oralen Einnahme bzw. Applikation mit zu geringen Wassermengen, zur Bildung von aufgequollenen Klumpen des Granulats kommen kann, die in den schlimmsten Fällen zu einem Verschluß der Speiseröhre oder aber sogar des Magen/Darm-Traktes führen können.

Auf dem Markt befindliche Laxativa auf Basis von Flohsamen oder indischem Flohsamen (Psyllium) oder deren Bestandteilen liefern oftmals keine ausreichende abführende Wirkung und bleiben nach Suspension in Wasser aufgrund der rasch einsetzenden Quellwirkung des Flohsamens nicht lange trinkfähig, so daß sie unmittelbar nach Herstellung der wäßrigen Suspension eingenommen werden müssen. Denn nach Suspension in Wasser gelieren diese Produkte bereits nach wenigen Minuten und bilden einen dicken, untrinkbaren Klumpen auf der Oberfläche. Zusätzlich erfolgt bei den meisten Präparaten sehr schnell mindestens eine zweischichtige Phasentrennung, welche die homogene Verteilung der wirksamen Komponenten in dem angerührten Präparat nicht mehr gewährleistet. Auch diese Präparate müssen mit reichlich Wasser eingenommen werden, weil ansonsten die Gefahr besteht, daß es zur Bildung von aufgequollenen Klumpen kommen kann, die in den schlimmsten Fällen zu einem Verschluß der Speiseröhre oder aber sogar des Magen-Darm-Traktes führen können. Weiterhin ist bei diesen Produkten auch der fade Geschmack und das künstliche Aussehen nachteilig.

Des weiteren ist in der auf die Anmelderin selbst zurückgehenden DE 103 46 083 A1 bzw. in den parallelen, zu derselben Patentfamilie gehörenden Dokumenten WO 2005/027948 A1 und US 2005/0053676 A1 ein Laxativum auf Basis einer pulverförmigen Zusammensetzung beschrieben, welches neben Plantagosamen (Plantaginis ovatae Semen) und/oder Plantagosamenschalen sowie mindestens einer anthranoiden Verbindung mit abführender Wirkung, insbesondere mindestens einem Sennosid, vorzugsweise in Form von anthranoid- bzw. sennosidhaltigen Pflanzen(bestand)teilen, außerdem mindestens ein polygalactomannanbasiertes Polysaccharid oder dessen Derivat, insbesondere Guaran (Guar-Gummi), enthält. Letzteres fungiert zum einen als (Co-)Stabilisator in einer wäßrigen Suspension der pulverförmigen Zusammensetzung und wirkt zum anderen, insbesondere aufgrund seiner Quellfähigkeit, synergistisch mit den anderen Komponenten der Zusammensetzung zusammen, indem es deren abführende Wirkung unterstützt, und erleichtert zudem die Verarbeitbarkeit der erfindungsgemäßen Zusammensetzung zu einem feinteiligen Pulver, welches in Wasser ohne weiteres dispergierbar ist. Jedoch ist bei der vorgenannten Zusammensetzung eine zusätzliche Komponente, nämlich das polygalactomannanbasiertes Polysaccharid oder dessen Derivat, erforderlich, um die gewünschten Eigenschaften zu erreichen. Auch kommen teilweise relativ hohe Dosierungen zum Einsatz. Zudem ist die vorgenannte Zusammensetzung in ihrer Natur als Pulver mit einem hohen Staubanteil behaftet.

In der gleichermaßen auf die Anmelderin selbst zurückgehenden US 4 511 561 A ist ein Laxans in Form eines Granulats beschrieben, welches neben Plantagosamen (Plantaginis ovatae Semen) und/oder Plantagosamenschalen sowie Sennesfrüchten auch noch Tragantgummi enthält.

Die US 5,320,847 A betrifft eine Zusammensetzung mit hohem Ballaststoffanteil, welche als Laxans eingesetzt werden kann, wobei die Zusammensetzung vermahlene Plantagosamenschalen, die mit einem wasserlöslichen schwachviskosen Pflanzengummi zur Stabilisierung agglomeriert werden, enthält, um eine trockene, rieselfähige, in Wasser suspendierbare diätetische Faserzusammensetzung zu formen. Die Zusammensetzung wird eingesetzt, um Obstipationen des Magen/Darm-Trakts durch die Aufnahme einer effektiven Menge der in Wasser dispergierten ballaststoffhaltigen Zusammensetzung entgegenzuwirken. Optional können dieser Zusammensetzung Sennoside in Form von Sennesblätterextrakt oder Pulver aus Sennesfrüchten zugefügt werden.

Die WO 2007/013093 A2 betrifft ebenfalls eine pharmazeutische Zusammensetzung zur Behandlung von Obstipationen des Magen/Darm-Trakts, wobei diese Zusammensetzung auf vermahlenen Plantagosamenschalen, Calciumsennosiden, Triphalaextrakt, einem Dispergiermittel sowie weiteren Additiven zur Verbesserung des Geschmacks und des optischen Eindrucks basiert. Die Verabreichung der pharmazeutischen Zusammensetzung erfolgt durch Dispersion der granulären Komposition in einer adäquaten Wassermenge.

Die US 5,232,699 A betrifft eine laxative Zusammensetzung, welche Plantagosamen und Sennoside enthält, wobei die Sennoside in einem genießbaren Lebensmittelfett dispergiert vorliegen und die Dispersion einen Schmelzpunkt zwischen 30 und 50 °C besitzt. Die beschriebenen Laxativa liegen insgesamt in Form von Gebäckwaffeln (*sandwich-type wafers* bzw. *coated wafers)* vor, indem Schichten der Sennosidfettdispersion und der samenschalenhaltigen Schichten angeordnet sind.

Die EP 0 387 933 A1 betrifft ballaststoffhaltige Kekse *(baked cookies*), welche unter anderem auch Plantagosamenschalen enthalten. Weitere Bestandteile dieses Gebäcks umfassen Lebensmittelfett, Mehl, Zucker und Wasser. Zusätzlich können andere Ballaststoffe als Plantagosamenschalen enthalten sein.

Die FR 2 646 352 A1 betrifft eine pharmazeutische Zusammensetzung, welche als Laxans einzusetzen ist und Plantagosamenschalen, Sennesfrüchteextrakt, Pflaumenextrakt sowie einen Stabilisator zur Realisierung der Dispergierbarkeit in Wasser enthält. Die pharmazeutische Zusammensetzung kann in Form eines Pulvers, in Form von Kapseln sowie optional in Form eines Granulats vorliegen.

Die WO 2008/080809 A2 betrifft eine laxative Zusammensetzung, welche pharmazeutisch verträgliche Additive in Kombination mit pharmazeutisch aktiven Ingredienzien, umfassend fein vermahlene Plantagosamen bzw. Plantagosamenschalen sowie vermahlene Sennesfrüchte oder Sennesfrüchteextrakt, in Form eines Pulvers mit einem Wassergehalt von weniger als 5 %, bezogen auf das Gewicht der gesamten Zusammensetzung, enthält.

Somit liegt der vorliegenden Erfindung die Aufgabe zugrunde, eine Zusammensetzung zur Verwendung als Laxativum bereitzustellen, welche die vorgeschilderten Nachteile zumindest weitgehend vermeidet.

Eine weitere Aufgabe der vorliegenden Erfindung ist die Bereitstellung einer als Laxativum verwendbaren Zusammensetzung, die einerseits eine gute abführende Wirkung zeigt, aber gleichzeitig auch eine erleichterte, komplikationsfreie Applikation ermöglicht, insbesondere ohne die vorgeschilderten Probleme bzw. Risiken.

Die Anmelderin hat nun überraschenderweise herausgefunden, daß sich das zuvor geschilderte Problem dadurch lösen läßt, daß man einerseits die Inhaltsstoffe der Sennesfrüchte in Form eines Sennesfrüchtetrockenextraktes bereitstellt und andererseits auf die Inkorporierung von Plantagosamenschalen verzichtet wird und ausschließlich Plantagosamen selbst eingesetzt wird.

Gegenstand der vorliegenden Erfindung - gemäß einem **ersten** Aspekt der vorliegenden Erfindung - ist somit eine pharmazeutische Zusammensetzung, insbesondere zur Verwendung als Laxans (Abführmittel), wobei die Zusammensetzung als Granulat vorliegt, wobei die Zusammensetzung in Kombination und in jeweils pharmazeutisch wirksamen Mengen
(A) 1 bis 15 Gew.-% Sennesfrüchtetrockenextrakt, bezogen auf die Zusammensetzung, wobei der Sennesfrüchtetrockenextrakt ein Droge/Extrakt-Verhältnis von mindestens 2 : 1 aufweist,
(B) 20 bis 90 Gew.-% Plantagosamen *(Plantaginis ovatae Semen),* bezogen auf die Zusammensetzung, wobei auf die Inkorporierung von Plantagosamenschalen verzichtet wird und ausschließlich Plantagosamen eingesetzt wird, und
(C) 3 bis 30 Gew.-% mindestens eines Granulatbildners, bezogen auf die Zusammensetzung,
enthält und
wobei die Zusammensetzung eine definierte Teilchengröße (Korngröße) und/oder Teilchengrößenverteilung (Korngrößenverteilung) aufweist derart, daß mehr als 99 Gew.-% der Teilchen der Zusammensetzung kleiner als 2.000 µm und mehr als 94 Gew.-% der Teilchen kleiner als 1.000 µm sind, wobei alle vorgenannten Gewichtsangaben jeweils auf das Trockengewicht der Gesamtzusammensetzung bezogen sind.

Die erfindungsgemäße pharmazeutische Zusammensetzung zeigt im Vergleich zu Zusammensetzungen des Standes der Technik, welche getrocknete und zerkleinerte Sennesfrüchte selbst einsetzen, infolge der Verwendung eines Sennesfrüchtetrockenextraktes eine verbesserte pharmakologische bzw. pharmazeutische Wirksamkeit bei gleichen Dosismengen aufgrund einer erhöhten Wirkstoffkonzentration im Sennesfrüchtetrockenextrakt (d. h. Konzentration an Hydroxyanthracenderivaten, insbesondere Sennosiden).

Infolge der Applikation von gegenüber dem Stand der Technik verringerten Einnahmemengen ist selbst bei unsachgemäßer Anwendung (insbesondere bei Applikation mit zu geringen Flüssigkeitsmengen bzw. sogar ohne Flüssigkeitsmengen) die Gefahr der Bildung von aufgequollenen Klumpen, welche in den schlimmsten Fällen zu einem Verschluß der Speiseröhre oder aber sogar des Magen/Darm-Traktes führen können, zumindest im wesentlichen ausgeschlossen.

Zudem ist die erfindungsgemäße pharmazeutische Zusammensetzung auch ohne die Anwesenheit eines zusätzlichen (Co-)Stabilisators stabil in Wasser suspendierbar bzw. dispergierbar bzw. löslich und bleibt eine wäßrige Suspension der erfindungsgemäßen Zusammensetzung über einen längeren Zeitraum stabil und somit trinkbar, d. h. die Suspension führt zu keiner vorzeitigen Phasentrennung und geliert auch nicht vorzeitig. Infolge des Verzichts auf Plantagosamenschalen wird die Suspendierbarkeit bzw. Dispergierbarkeit zusätzlich verbessert und das Problem bzw. die Gefahr der Klumpenbildung zusätzlich reduziert.

Um eine gute Wirkstoffkonzentration zu erhalten, enthält der erfindungsgemäß eingesetzte Sennesfrüchtetrockenextrakt üblicherweise einen Gehalt an Hydroxyanthracenderivaten, bezogen auf den Sennesfrüchtetrockenextrakt und insbesondere berechnet als Sennosid B, von mindestens 3 Gew.-%, insbesondere mindestens 5 Gew.-%, vorzugsweise mindestens 7 Gew.-%, besonders bevorzugt mindestens 8 Gew.-%. Dennoch kann es einzelfallbedingt oder anwendungsbezogen erforderlich sein, von den vorgenannten Gehalten an Hydroxyanthracenderivaten, insbesondere Sennosiden, abzuweichen, ohne daß der Rahmen der vorliegenden Erfindung verlassen ist.

Alle vorgenannten und noch folgenden Gewichtsangaben sind jeweils auf das Trockengewicht der erfindungsgemäßen Gesamtzusammensetzung bezogen, sofern nicht ausdrücklich Gegenteiliges vermerkt ist.

Üblicherweise enthält der erfindungsgemäß eingesetzte Sennesfrüchtetrockenextrakt einen Gehalt an Hydroxyanthracenderivaten, insbesondere Sennosiden, bezogen auf den Sennesfrüchtetrockenextrakt und insbesondere berechnet als Sennosid B, im Bereich von 3 bis 30 Gew.-%, insbesondere 5 bis 25 Gew.-%, vorzugsweise 7 bis 20 Gew.-%, besonders bevorzugt 8 bis 15 Gew.-%, ganz besonders bevorzugt 8 bis 12 Gew.-%.

Das Droge/Extrakt-Verhältnis des eingesetzten Sennesfrüchtetrockenextraktes kann in relativ breiten Bereichen variieren: Üblicherweise weist der erfindungsgemäß verwendete Sennesfrüchtetrockenextrakt ein Droge/Extrakt-Verhältnis von mindestens 2 : 1, insbesondere mindestens 2,5 : 1, vorzugsweise mindestens 3 : 1, auf. Im allgemeinen liegt das Droge/Extrakt-Verhältnis des eingesetzten Sennesfrüchtetrockenextraktes im Bereich von 2 : 1 bis 10: 1, insbesondere 2,5: 1 bis 8 : 1, vorzugsweise 3 : 1 bis 6 : 1.

Besonders gute Ergebnisse werden erzielt, wenn der Sennesfrüchtetrockenextrakt auf Basis von Früchten der Alexandriner-Sennapflanze und/oder der Tinnevelly-Sennapflanze ausgebildet ist, wobei auch Mischungen der beiden vorgenannten Sennapflanzen in beliebigen Mischungsverhältnissen zum Einsatz kommen können. Üblicherweise ist der erfindungsgemäß eingesetzte Sennesfrüchtetrockenextrakt durch Extraktion von Früchten der Alexandriner-Sennapflanze und/oder der Tinnevelly-Sennapflanze erhältlich. Auf das diesbezügliche Extraktionsverfahren wird diesbezüglich noch im Detail eingegangen werden, so daß sich diesbezügliche Ausführungen an dieser Stelle erübrigen.

Vorteilhafterweise ist der erfindungsgemäß eingesetzte Sennesfrüchtetrockenextrakt wasserlöslich ausgebildet. Dies erleichtert die Applikation, da der Sennesfrüchtetrockenextrakt beim Anrühren mit Wasser dann in Lösung geht und dort stabil verbleibt.

Im allgemeinen enthält der erfindungsgemäß eingesetzte Sennesfrüchtetrockenextrakt ein Gemisch verschiedener anthranoider Verbindungen auf Basis von Hydroxyanthracenderivaten, insbesondere Sennosiden.

Üblicherweise sind diese Hydroxyanthracenderivate, insbesondere Sennoside, ausgewählt aus der Gruppe der folgenden Verbindungen der allgemeinen Formel (I): 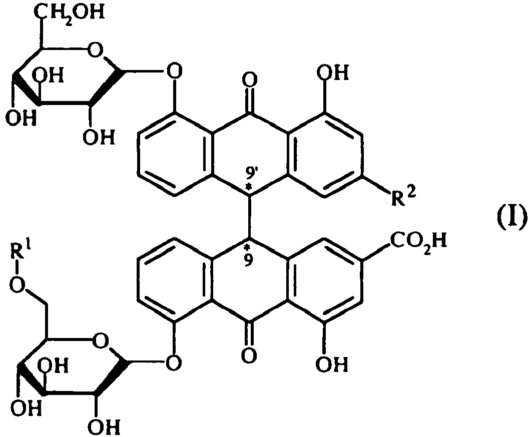 wobei in der allgemeinen Formel (I)
- der Rest R¹ Wasserstoff oder eine Gruppe -CO-CO₂H darstellt,
- der Rest R² eine Gruppe -CO₂H oder -CH₂OH darstellt, jedoch mit der Maßgabe, daß, wenn R¹ eine Gruppe -CO-CO₂H bezeichnet, R² eine Gruppe -CO₂H darstellt,
- die mit dem Zeichen "*" gekennzeichneten Kohlenstoffatome in 9- und 9'-Position des Anthrongerüstes Chiralitätszentren darstellen,
sowie deren Mischungen und/oder Stereoisomeren, insbesondere Enantiomeren und/oder Diastereoisomeren, und/oder Derivaten der vorgenannten Verbindungen.

Gemäß einer erfindungsgemäß bevorzugten Ausführungsform sind die Hydroxyanthracenderivate, insbesondere Sennoside, ausgewählt aus der Gruppe der folgenden Verbindungen der allgemeinen Formel (I) und deren Mischungen:

| Verbindung | R¹ | R² | 9-9' |
|---|---|---|---|
| (I A) | -H | -CO₂H | R*,R* (threo) |
| (I B) | -H | -CO₂H | R*.S* (erythro) |
| (I C) | -H | -CH₂OH | R*,R* (threo) |
| (I D) | -H | -CH₂OH | R*,S* (erythro) |
| (I E) | -CO-CO₂H | -CO₂H | R*,R* (threo) |
| (I F) | -CO-CO₂H | -CO₂H | R*,S* (erythro) |

Zur Erzielung einer guten pharmazeutischen bzw. pharmakologischen Wirkung ist es vorteilhaft, wenn die erfindungsgemäße pharmazeutische Zusammensetzung den Sennesfrüchtetrockenextrakt in Mengen von mindestens 1 Gew.-%, insbesondere mindestens 2 Gew.-%, vorzugsweise mindestens 3 Gew.-%, besonders bevorzugt mindestens 4 Gew.-%, ganz besonders bevorzugt mindestens 5 Gew.-%, bezogen auf die Zusammensetzung, enthält. Im allgemeinen enthält die erfindungsgemäße pharmazeutische Zusammensetzung den Sennesfrüchtetrockenextrakt in Mengen von 1 bis 15 Gew.-%, insbesondere 2 bis 10 Gew.-%, vorzugsweise 3 bis 8 Gew.-%, besonders bevorzugt 4 bis 7 Gew.-%, ganz besonders bevorzugt 4,5 bis 6,5 Gew.-%, bezogen auf die Zusammensetzung.

Im allgemeinen ist die erfindungsgemäße pharmazeutische Zusammensetzung durch einen Gesamtgehalt an Hydroxyanthracenderivaten, insbesondere Sennosiden, von mindestens 0,1 Gew.-%, insbesondere mindestens 0,2 Gew.-%, vorzugsweise mindestens 0,3 Gew.-%, besonders bevorzugt mindestens 0,4 Gew.-%, ganz besonders bevorzugt mindestens 0,5 Gew.-%, bezogen auf die Zusammensetzung und insbesondere berechnet als Sennosid B, gekennzeichnet. Üblicherweise beträgt der Gesamtgehalt an Hydroxyanthracenderivaten, insbesondere Sennosiden, in der erfindungsgemäßen Zusammensetzung, bezogen auf die Zusammensetzung und insbesondere berechnet als Sennosid B, 0,1 bis 2 Gew.-%, insbesondere 0,2 bis 1 Gew.-%, vorzugsweise 0,3 bis 0,8 Gew.-%, besonders bevorzugt 0,4 bis 0,7 Gew.-%. Dennoch kann es einzelfallbezogen oder anwendungsbedingt vorteilhaft oder erforderlich sein, von den vorgenannten Mengen abzuweichen, ohne daß der Rahmen der vorliegenden Erfindung verlassen ist.

Was die Plantagosamenkomponente in der erfindungsgemäßen Zusammensetzung anbelangt, so beträgt deren Gehalt zur Erzielung einer guten Wirksamkeit erfindungsgemäß mindestens 20 Gew.-%, insbesondere mindestens 30 Gew.-%, vorzugsweise mindestens 40 Gew.-%, besonders bevorzugt mindestens 50 Gew.-%, ganz besonders bevorzugt mindestens 60 Gew.-%, bezogen auf die Zusammensetzung. Üblicherweise liegt der Gehalt an Plantagosamen in der erfindungsgemäßen Zusammensetzung im Bereich von 20 bis 90 Gew.-%, insbesondere 30 bis 90 Gew.-%, vorzugsweise 40 bis 85 Gew.-%, besonders bevorzugt 50 bis 80 Gew.-%, ganz besonders bevorzugt 65 bis 75 Gew.-%.

Die erfindungsgemäße pharmazeutische Zusammensetzung liegt in Form eines Granulats vor. Hiermit ist eine Reihe von Vorteilen verbunden. Die Granulatform erleichtert die Applikation in mehrfacher Hinsicht: Zum einen ist die Dosierung erleichtert (z. B. ermöglicht die Form eines Granulats eine Teelöffeldosierung), und zum anderen erhöht die Granulatform einerseits die Lagerstabilität der Zusammensetzung und andererseits deren Suspendierbarkeit bzw. Lösbarkeit in Wasser für die betreffende Applikation (d. h. genauer gesagt gehen die Psylliumsamenbestandteile in Suspension, während die übrigen Bestandteile, insbesondere der Sennesfrüchtetrockenextrakt und gegebenenfalls weitere Inhaltsstoffe, wie z. B. Granulatbildner, in Lösung treten). Auch läßt sich auf diese Weise die Stabilität der hergestellten Suspension bzw. Lösung erhöhen. Ferner wird durch die Granulatform sichergestellt, daß die erfindungsgemäße Zusammensetzung staubfrei ausgebildet ist, was insbesondere die Anwendbarkeit erleichtert und die Risiken (z. B. Inhalation bei unsachgemäßer Anwendung) hierbei minimiert.

Es war für den Fachmann nicht zu erwarten, daß die erfindungsgemäße Zusammensetzung sich überhaupt zu einem Granulat verarbeiten läßt, da betreffende Versuche mit zerkleinerten Bestandteilen der Sennesfrüchte und Psylliumbestandteilen zu Verklumpungen und einem vorzeitigen Quellen führen. Erst durch die Verwendung des Sennesfrüchtetrockenextraktes gelingt es, die erfindungsgemäße Zusammensetzung in die Granulatform zu überführen, was von vornherein nicht absehbar war.

Die pharmazeutische Zusammensetzung nach der vorliegenden Erfindung enthält außerdem (C) mindestens einen Granulatbildner. Der Granulatbildner - synonym auch als Granulierhilfsmittel, Granuliersubstanz etc. bezeichnet - dient gewissermaßen als Gerüstsubstanz bzw. sozusagen als "Klebstoff" für die zu granulierenden Inhaltsstoffe. Darauf wird nachfolgend im Rahmen der Beschreibung des erfindungsgemäßen Herstellungsverfahrens noch im Detail eingegangen werden, so daß sich weitergehende Ausführungen an dieser Stelle erübrigen.

Die pharmazeutische Zusammensetzung nach der Erfindung enthält den oder die Granulatbildner in Mengen von 3 bis 30 Gew.-%, insbesondere 4 bis 25 Gew.-%, vorzugsweise 5 bis 20 Gew.-%, besonders bevorzugt 6 bis 15 Gew.-%, ganz besonders bevorzugt 7 bis 10 Gew.-%, bezogen auf die Zusammensetzung.

Für diesen Zweck geeignete Granulatbildner sind dem Fachmann als solche bekannt. Beispielsweise kann der erfindungsgemäß eingesetzte Granulatbildner ausgewählt sein aus der Gruppe von Stärkederivaten (z. B. Stärkeabbauprodukten, insbesondere Dextrinen und Maltodextrinen, vorzugsweise Maltodextrinen), Cellulose und Cellulosederivaten (z. B. Ethylcellulose), Poly(meth)acrylsäuren und Poly(meth)acrylaten (z. B. Eudragite· oder Carbomere, wie z. B. Carbopol·), Gelatine, Polyvinylpyrrolidon (PVP), Polyalkylenglykolen (z. B. Polyethylenglykol, wie z. B. Movicol·), Dextrose (D-Glucose), Lactose, Maltose und Zuckeraustauschstoffen (z. B. Fructose und Zuckeralkoholen, wie z. B. Mannit, Xylit, Sorbit (D-Glucit), Isomaltit (Isomalt), Maltit und Lactit) sowie deren Mischungen.

Als Granulatbildner besonders bevorzugt sind Stärkederivate, insbesondere Stärkeabbauprodukte, wie insbesondere Dextrine und Maltodextrine, besonders bevorzugt Maltodextrine. Für weitergehende Einzelheiten zu Dextrinen und Maltodextrinen kann insbesondere verwiesen werden auf Römpp Chemielexikon, 10. Auflage, Georg Thieme Verlag Stuttgart/New York, Band 2, 1997, Seite 928, Stichwort "Dextrine", und Band 4, 1998, Seite 2513, Stichwort: "Maltodextrine", und auf Römpp Lexikon Lebensmittelchemie, Georg Thieme Verlag, Stuttgart/New York, 9. Auflage, 1995, Seite 213, Stichwort: "Dextrin", und Seite 518, Stichwort: "Maltodextrine", sowie die dort jeweils referierte Literatur, deren jeweiliger Inhalt hiermit durch Bezugnahme eingeschlossen ist.

Der erfindungsgemäß eingesetzte Granulatbildner sollte vorteilhafterweise wasserlöslich oder zumindest aber wasserdispergierbar, vorzugsweise wasserlöslich, ausgebildet sein, um einerseits bei der Granulierung als Lösung eingesprüht werden zu können und um sich andererseits bei der peroralen Applikation mit Wasser in der Trinkflüssigkeit lösen zu lassen.

Ganz besonders bewährt haben sich als Granulatbildner Maltodextrine. Maltrodextrine eignen sich als besonders stabile Gerüstsubstanzen für den Granulataufbau und gewährleisten eine gute Adhäsion bzw. Verklebung der einzelnen Inhaltsstoffe und eignen sich zudem bei der Aufbereitung bzw. Applikation, da sie insbesondere eine besonders gute und schnelle Löslichkeit bzw. Suspendierbarkeit der entsprechenden Trinkflüssigkeit, insbesondere auch im kalten Zustand, gewährleisten.

Wie zuvor geschildert und nachfolgend im Rahmen des erfindungsgemäßen Herstellungsverfahren noch beschrieben, ist für den Fall, daß die erfindungsgemäße Zusammensetzung als Granulat ausgebildet ist, das Granulat vorteilhafterweise durch Wirbelschichtgranulierung erhältlich.

Neben den vorgenannten Wirk- und Inhaltsstoffen kann die erfindungsgemäße Zusammensetzung außerdem mindestens ein weiteres Additiv, mindestens einen weiteren Inhaltsstoff und/oder einen weiteren Zusatzstoff enthalten. Letztere können insbesondere ausgewählt sein aus der Gruppe von Farbstoffen wie natürlichen oder naturidentischen Farbstoffen, Geschmacksmitteln, Geschmacksverstärkern und Aromastoffen, Süßungsmitteln, Säuerungsmitteln, Konservierungsmitteln, Stabilisatoren und Costabilisatoren, Elektrolyten, Mineralien und Mineralstoffen, Vitaminen, Füllstoffen, Fließmitteln und Verarbeitungshilfsmitteln sowie Mischungen der vorgenannten Verbindungen.

Erfindungsgemäß weist die pharmazeutische Zusammensetzung nach der vorliegenden Erfindung in Form eines Granulats eine definierte Teilchengröße (synonym als Korngröße oder Partikelgröße bezeichnet) und/oder eine definierte Teilchengrößenverteilung (synonym als Korngrößenverteilung oder Partikelgrößenverteilung bezeichnet) auf. Die Teilchengrößen (Korngrößen bzw. Partikelgrößen) lassen sich durch an sich bekannte Methoden bestimmen, so z. B. durch Siebanalyse, granulometrisch, durch Lichtbeugung, mikroskopisch etc. Die nachfolgenden Teilchengrößenangaben und Teilchengrößenverteilungsangaben beziehen sich insbesondere auf Werte aus der Siebanalyse gemäß Ph. Eur. (Pharmacopoea Europea), 5. Ausgabe, Grundwerk 2005, Seite 298, Kapitel 2.9.12 "Siebanalyse" (ISBN-Nr.: 3-7692-3638-6).

Im allgemeinen ist die pharmazeutische Zusammensetzung nach der vorliegenden Erfindung, insbesondere in Form des Granulats, zumindest im wesentlichen staubfrei ausgebildet. Dies hat nicht nur herstellungstechnische Vorteile, sondern auch Vorteile in bezug auf die Anwendung, da beim Einrühren der erfindungsgemäßen Zusammensetzung in die Trinkflüssigkeit keine Verklumpung eintritt und zudem eine Inhalation auch bei unsachgemäßer Applikation ausgeschlossen ist und im Lagerzustand keine vorzeitige Quellung des Plantagosamen erfolgt.

Üblicherweise weist die pharmazeutische Zusammensetzung nach der vorliegenden Erfindung in Form des Granulats eine mittlere Teilchengröße (mittlere Korngröße) im Bereich von 50 bis 400 µm, insbesondere 50 bis 300 µm, vorzugsweise 75 bis 275 µm, auf. Dies ermöglicht zum einen eine problemlose Herstellung, und zum anderen führt dies zu einer guten Lagerstabilität und zu einer guten Einrührbarkeit in die Applikationsflüssigkeit.

Gemäß einer bevorzugten Ausführungsform weist der Hauptmassenanteil der pharmazeutischen Zusammensetzung nach der vorliegenden Erfindung (insbesondere in Form des Granulats), vorzugsweise mehr als 55 Gew.-%, insbesondere mehr als 60 Gew.-%, bevorzugt mehr als 65 Gew.-%, besonders bevorzugt mehr als 70 Gew.-%, ganz besonders bevorzugt mehr als 75 Gew.-% der Teilchen der Zusammensetzung, Korngrößen im Bereich von 50 bis 1.000 µm, bevorzugt mit einem Maximum der Verteilung insbesondere bei Teilchengrößen im Bereich von 65 bis 500 µm, vorzugsweise 125 bis 450 µm, auf.

Gemäß einer erfindungsgemäß besonders bevorzugten Ausführungsform weist die pharmazeutische Zusammensetzung nach der vorliegenden Erfindung, insbesondere in Form des Granulats, folgende Teilchengrößenverteilung (Korngrößenverteilung) auf:
- mehr als 99 Gew.-%, vorzugsweise 100 Gew.-%, der Teilchen der Zusammensetzung kleiner als 2.000 µm; und/oder
- mehr als 94 Gew.-%, insbesondere mehr als 95 Gew.-%, vorzugsweise mehr als 96 Gew.-%, der Teilchen der Zusammensetzung kleiner als 1.000 µm; und/oder
- mehr als 50 Gew.-%, insbesondere mehr als 55 Gew.-%, vorzugsweise mehr als 60 Gew.-%, besonders bevorzugt mehr als 65 Gew.-%, ganz besonders bevorzugt mehr als 70 Gew.-%, der Teilchen der Zusammensetzung kleiner als 500 µm; und/oder
- weniger als 10 Gew.-%, insbesondere weniger als 5 Gew.-%, vorzugsweise weniger als 4 Gew.-%, besonders bevorzugt weniger als 3 Gew.-%, ganz besonders bevorzugt weniger als 1 Gew.-%, der Teilchen der Zusammensetzung kleiner als 63 µm;
wobei alle vorgenannten Gewichtsangaben jeweils auf das Trockengewicht der Gesamtzusammensetzung bezogen sind.

Gemäß einer erfindungsgemäß ganz besonders bevorzugten Ausführungsform weist die pharmazeutische Zusammensetzung nach der vorliegenden Erfindung, insbesondere in Form des Granulats, folgende Teilchengrößenverteilung (Korngrößenverteilung) (Siebspektrum gemäß Siebanalyse) auf:
- Teilchengrößen > 2.000 µm:
   weniger als 1 Gew.-%, insbesondere 0 Gew.-%;
- 2.000 µm ≤ Teilchengrößen > 1.000 µm:
   0,5 bis 10 Gew.-%, insbesondere 1 bis 10 Gew.-%, vorzugsweise 1 bis 6 Gew.-%;
- 1.000 µm ≤ Teilchengrößen > 500 µm:
   15 bis 45 Gew.-%, insbesondere 20 bis 40 Gew.-%, vorzugsweise 25 bis 40 Gew.-%;
- 500 µm ≤ Teilchengrößen > 250 µm:
   35 bis 60 Gew.-%, insbesondere 40 bis 55 Gew.-%, vorzugsweise 40 bis 50 Gew.-%;
- 250 µm ≤ Teilchengrößen > 125 µm:
   5 bis 30 Gew.-%, insbesondere 10 bis 25 Gew.-%, vorzugsweise 10 bis 20 Gew.-%;
- 125 µm ≤ Teilchengrößen > 63 µm:
   0,5 bis 5 Gew.-%, insbesondere 0,5 bis 4 Gew.-%, vorzugsweise 1 bis 3 Gew.-%;
- 63 µm ≤ Teilchengrößen > 45 µm:
   0 bis 3 Gew.-%, insbesondere 0 bis 1 Gew.-%, vorzugsweise 0 Gew.-%;
wobei alle vorgenannten Gewichtsangaben jeweils auf das Trockengewicht der Gesamtzusammensetzung bezogen sind und mit der Maßgabe, daß die Summe der Gewichtsprozentangaben 100 Gew.-% ergibt (d. h. die Gewichtsanteile der Teilchen mit den einzelnen Teilchengrößenbereichen sind derart zu kombinieren, daß sie in Summe 100 Gew.-% ergeben).

Weiterhin weist die pharmazeutische Zusammensetzung nach der vorliegenden Erfindung vorteilhafterweise eine Schüttdichte im Bereich von 250 bis 650 g/l, insbesondere 300 bis 600 g/l, vorzugsweise 350 bis 550 g/l, besonders bevorzugt 400 bis 500 g/l, auf. Dies ermöglicht eine gute, insbesondere teelöffelweise Dosierung der erfindungsgemäßen pharmazeutischen Zusammensetzung, vorteilhafterweise in Form eines Granulats.

Im Rahmen der vorliegenden Erfindung wird somit eine pharmazeutische Zusammensetzung, insbesondere in Form eines Granulats, bereitgestellt, welche die eingangs geschilderten Nachteile des Standes der Technik in effizienter Weise vermeidet oder aber wenigstens abschwächt.

Durch die Verwendung eines Sennesfrüchtetrockenextrakts wird ein im Vergleich zum Stand der Technik signifikant höherer Hydroxyanthracenderivatgehalt, insbesondere Sennosidgehalt, bei gleicher Dosiermenge erreicht, was zu dem Vorteil führt, daß zur Erzielung einer vergleichbaren Wirkung geringere Mengen der Zusammensetzung appliziert werden müssen als im Stand der Technik; dies ist nicht nur aus physiologischer Sicht wünschenswert, sondern minimiert auch das Risiko von Gefahren bei einer unsachgemäßen Anwendung (z. B. keine Gefahr der Klumpenbildung bei einer Einnahme mit zu wenig Flüssigkeit etc.). Auch herstellungs- und anwendungstechnisch bietet die Verwendung eines Sennesfrüchtetrockenextraktes eine Reihe von Vorteilen: Durch die Verwendung eines Sennesfrüchtetrockenextraktes läßt sich die Herstellung der Gesamtzusammensetzung deutlich erleichtern; der Sennesfrüchtetrockenextrakt läßt sich insbesondere besser und lagerstabiler granulieren als die getrockneten und zerkleinerten Sennesfrüchte. Ein weiterer Vorteil der Verwendung des Sennesfrüchtetrockenextraktes, insbesondere im Rahmen der erfindungsgemäßen Zusammensetzung in Form eines Granulats, ist die verbesserte Löslichkeit bzw. Suspendierbarkeit bei der peroralen Anwendung; insbesondere läßt sich die pharmazeutische Zusammensetzung in eine stabile Suspension bzw. Lösung überführen, welche über mehrere Stunden ihre Konsistenz im wesentlichen nicht verändert und somit über mehrere Stunden anwendungsbereit bleibt. So ist der erfindungsgemäß eingesetzte Sennesfrüchtetrockenextrakt wasserlöslich. Infolge der staubfreien Ausbildung der erfindungsgemäßen Zusammensetzung sind sowohl herstellungstechnisch als auch anwendungsbezogene Vorteile verbunden.

Im Ergebnis ist es im Rahmen der vorliegenden Erfindung gelungen, eine pharmazeutische Zusammensetzung bereitzustellen, die gegenüber dem Stand der Technik deutlich verbessert ist.

Gegenstand der vorliegenden Erfindung - gemäß einem **zweiten** Aspekt der vorliegenden Erfindung - ist ein Verfahren zur Herstellung der zuvor beschriebenen pharmazeutischen Zusammensetzung nach der vorliegenden Erfindung. Bei dem erfindungsgemäßen Herstellungsverfahren werden die zuvor definierten Wirk- und/oder Inhaltsstoffe der erfindungsgemäßen Zusammensetzung, insbesondere (A) der Sennesfrüchtetrockenextrakt und (B) der Plantagosamen (Plantaginis ovatae Semen) sowie gegebenenfalls weitere Bestandteile der Zusammensetzung, wie sie zuvor definiert worden sind, gegebenenfalls nach vorangehender Einstellung der Teilchengrößen, insbesondere mittels Zerkleinerung, einer Granulierung, vorzugsweise im Wirbelschichtverfahren, in Gegenwart (C) mindestens eines zuvor definierten Granulatbildners unterzogen. Bezüglich der Mengenanteile und Konzentrationen der einzelnen Wirk- und/oder Inhaltsstoffe kann auf die vorangehenden Ausführungen zu der erfindungsgemäßen Zusammensetzung verwiesen werden, welche in bezug auf das erfindungsgemäße Herstellungsverfahren entsprechend gelten, so daß sich unnötige Wiederholungen erübrigen. Die Granulierung wird üblicherweise außerdem in Gegenwart einer Granulierflüssigkeit, im allgemeinen Wasser, durchgeführt; wie dem Fachmann als solches bekannt, wird die Granulierflüssigkeit mit einer bestimmten Sprührate eingetragen, wie nachfolgend noch beschrieben.

Dabei wird üblicherweise derart vorgegangen, daß zunächst eine Pulvermischung der Wirk- bzw. Inhaltsstoffe der erfindungsgemäßen Zusammensetzung hergestellt wird, wobei es vorteilhaft ist, die Teilchengrößen für die nachfolgende Granulierung entsprechend einzustellen. Vorteilhafterweise werden in der Pulverausgangsmischung die Teilchengrößen unterhalb von 200 µm, vorzugsweise unterhalb von 150 µm, eingestellt. Nachfolgend wird dann die Pulvermischung granuliert, vorzugsweise mittels Wirbelschichtgranulierung, wobei üblicherweise mit einer Zuluftrate, insbesondere einer maschinenabhängigen Zuluftrate, von etwa 200 bis 300 m³/h fluidisiert wird und nach Erreichen einer geeigneten Zulufttemperatur mit dem Einsprühen der Feuchtigkeit, insbesondere Wasser bzw. Wasserdampf, begonnen wird. Grundsätzlich ist es möglich, alle Wirk- bzw. Inhaltsstoffe der erfindungsgemäßen Zusammensetzung in der pulverförmigen Ausgangsmischung vorzulegen. Alternativ ist es auch möglich - und dies ist erfindungsgemäß bevorzugt-, nur einen Teil der Wirk- und Inhaltsstoffe, im allgemeinen zumindest den Sennesfrüchtetrockenextrakt und die zerkleinerten Plantagosamen, in der pulverförmigen Mischung vorzulegen, dagegen andere Bestandteile bzw. Inhaltsstoffe, insbesondere sofern sie wasserlöslich sind, in der Granulierflüssigkeit zu lösen und erst im Rahmen des Granuliervorgangs mit den übrigen Bestandteilen zu den betreffenden Granulaten zusammenzubringen.

Für die Durchführung einer effizienten Granulierung wird also die Granulierung in Gegenwart mindestens eines zuvor definierten Granulatbildners durchgeführt, d. h. üblicherweise liegt der Granulatbildner bereits in der pulverförmigen Ausgangsmischung vor oder aber wird - in erfindungsgemäß bevorzugter Weise - in der einzutragenden Granulierflüssigkeit gelöst.

Im allgemeinen erfolgt die Granulierung bei einer geeigneten Sprührate (ml/min), z. B. bei einer mittleren eingestellten Sprührate von 10 bis 100 ml Granulierflüssigkeit pro Minute, und/oder über eine Granulierdauer von insgesamt 30 bis 300 Minuten und/oder bei einer Granuliertemperatur im Bereich von 30 bis 150 °C, insbesondere 30 bis 100 °C (z. B. maschinenabhängig beispielsweise auch gegebenenfalls bei hiervon abweichenden, insbesondere höheren Temperaturen).

Durch Einstellung bzw. Steuerung der zuvor beschriebenen Granulierbedingungen und die Granulometrie der Ausgangsteilchen gelingt es, in effizienter Weise die Teilchengrößen und Teilchengrößenverteilung des Endprodukts bzw. des Granulats gezielt zu steuern und die gewünschte Granulometrie, wie zuvor definiert, einzustellen.

Für weitergehende Einzelheiten zu Granulierverfahren, insbesondere Wirbelschichtverfahren (Wirbelbettverfahren), im allgemeinen kann auf die allgemeine Fachliteratur verwiesen werden, beispielsweise auf Römpp Chemielexikon, Georg Thieme Verlag, Stuttgart/New York, 10. Auflage, z. B. die Stichwörter: "Granulate", "Prillen" und "Wirbelschichtverfahren" sowie die dort referierte Literatur.

Es ist vollkommen überraschend, daß sich die erfindungsgemäße Zusammensetzung in der zuvor geschilderten Weise in ein Granulat überführen läßt, da grundsätzlich eine Verklumpung und ein vorzeitiges Quellen des Plantagosamens zu erwarten gewesen wäre. Überraschenderweise treten diese Phänomene aber unter den vorgenannten Granulierbedingungen nicht auf, und es läßt sich in effizienter Weise ein Granulat erzeugen.

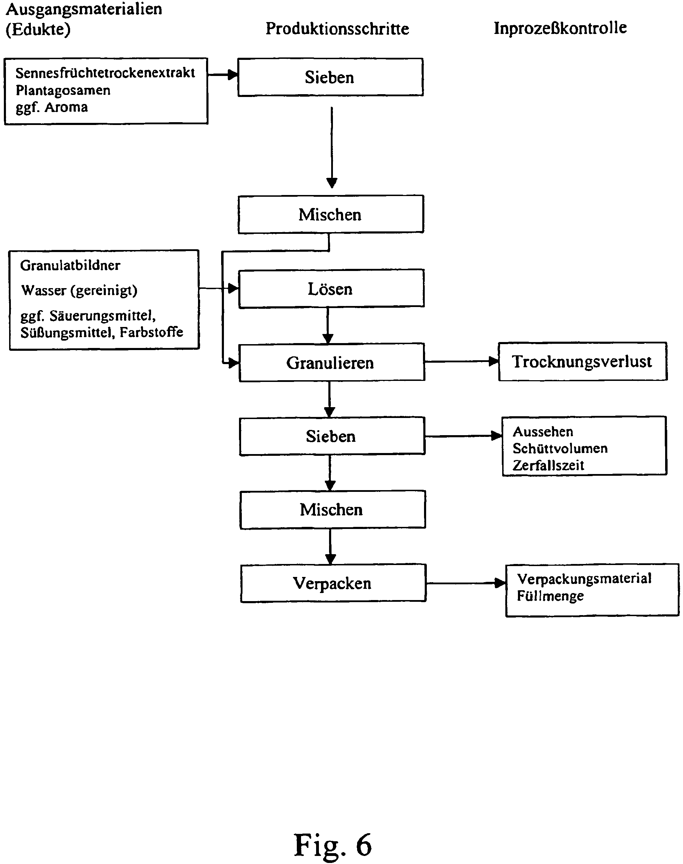

Fig. 6 zeigt in Form eines Fließschemas in einer schematischen Übersicht des erfindungsgemäßen Verfahrens gemäß einer typischen Ausführungsform, wie sie zuvor beschrieben worden ist und nachfolgend auch noch in den Ausführungsbeispielen wiedergegeben ist.

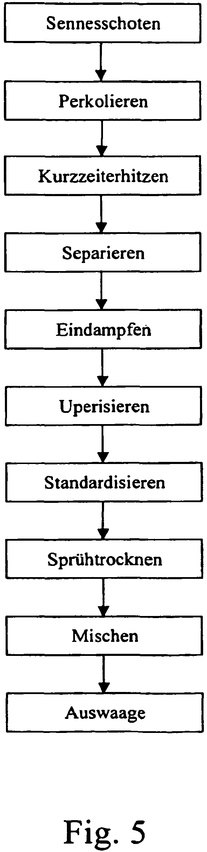

Die Herstellung des erfindungsgemäß eingesetzten Sennesfrüchtetrockenextrakts - synonym auch als Sennesschotentrockenextrakt bezeichnet - erfolgt im allgemeinen derart, daß geeignet zerkleinerte Sennesschoten bzw. Sennesfrüchte auf Basis von Alexandriner-Sennesfrüchten und/oder Tinnevelly-Sennesfrüchten (entweder jeweils sortenrein oder im beliebigen Mischungsverhältnis) in Perkolatoren überführt werden, welche mit kaltem Wasser gefüllt, in Reihe geschaltet und aufsteigend perkoliert werden. Nachfolgend wird das Perkolat im Dünnextraktbehälter gesammelt und anschließend unter Kurzzeiterhitzung erhitzt. Die ausgefallenen Feststoffe werden im Separator abgetrennt. Der dünne Extrakt wird mit Hilfe eines Vakuumfallstromverdampfers eingeengt. Der eingeengte Extrakt wird anschließend in einer Uperisationsanlage uperisiert. Die Sprühlösung wird dann mit Kohlendioxid versetzt und in einem Sprühturm getrocknet. Das Sprühprodukt wird in einem Mischer, insbesondere in einem Konusschneckenmischer, gemischt und - gegebenenfalls nach Zwischenlagerung - der nachfolgenden Herstellung der erfindungsgemäßen Zusammensetzung zugeführt. Der erfindungsgemäß eingesetzte Sennesfrüchtetrockenextrakt ist wasserlöslich ausgebildet. Eine schematische Übersicht des Ablaufs der Herstellung des Sennesfrüchtetrockenextraktes ist in Fig. 5 wiedergegeben.

Für weitergehende Details zu dem erfindungsgemäßen Herstellungsverfahren kann auch auf die Ausführungsbeispiele verwiesen werden.

Wie zuvor beschrieben, eignet sich die Zusammensetzung nach der vorliegenden Erfindung insbesondere zur Anwendung als Laxativum (Abführmittel, Laxans).

Gleichermaßen eignet sich somit die erfindungsgemäße Zusammensetzung zur Verwendung als Laxativum (Laxans, Abführmittel) bzw. zur Herstellung eines Arzneimittels oder einer pharmazeutische Zusammensetzung zur Förderung und/oder Erleichterung und/oder Regulierung der Darmentleerung und/oder der Darmtätigkeit.

Im Rahmen der zuvor beschriebenen Verwendung läßt sich die pharmazeutische Zusammensetzung nach der vorliegenden Erfindung insbesondere zur prophylaktischen oder kurativen Behandlung von Erkrankungen, bei denen eine leichte Defäkation mit leichtem Stuhl erwünscht ist, insbesondere bei Hämorrhoidalerkrankungen, Zuständen nach rektal-analen operativen Eingriffen und vor und nach operativen Eingriffen des Bauchraums sowie bei Obstipationen, oder zur Erleichterung der Darmpassage und Defäkation, insbesondere zur Linderung der Nebenwirkungen bei Therapie mit obstipationsverursachenden Medikamenten, einsetzen.

Im Rahmen der zuvor beschriebenen Verwendung wird die erfindungsgemäße Zusammensetzung oral eingenommen, vorzugsweise als wäßrige Suspension oder Lösung, insbesondere in Einzeldosen von 1 bis 5 g, insbesondere 2 bis 4 g, vorzugsweise 2,5 bis 3,5 g. Übliche Tagesdosen liegen im Bereich von insgesamt von 1 bis 50 g, vorzugsweise 1 bis 20 g, besonders bevorzugt 3 bis 10 g, der erfindungsgemäßen Zusammensetzung. Infolge der Bereitstellung der Sennoside in Form eines Sennesfrüchtetrockenextraktes gelingt es im Rahmen der erfindungsgemäßen Zusammensetzung, sowohl die Einzeldosen als auch die Tagesdosen gegenüber den Zusammensetzungen des Standes der Technik, welche getrocknete und pulverisierte Sennesfrüchte einsetzen, signifikant zu reduzieren, üblicherweise um bis zu 50 % oder sogar mehr.

Zu Zwecken der vereinfachten Applikation kann die erfindungsgemäße Zusammensetzung in Verpackungseinheiten (z. B. Sachets) für die entsprechenden Einzeldosen bereitgestellt werden (z. B. Sachets zu 1 bis 5 g). Aufgrund der guten Dosierbarkeit der erfindungsgemäßen Zusammensetzung, insbesondere in Form des Granulats, kann die erfindungsgemäße Zusammensetzung aber auch in Großgebinden (z. B. Dosen mit 40 bis 1.000 g) bereitgestellt werden, da die erfindungsgemäße Zusammensetzung eine einfache Teelöffeldosierung ermöglicht.

Auch eignet sich die erfindungsgemäße Zusammensetzung für ein Verfahren zur Behandlung des menschlichen Körpers, insbesondere zu Zwecken der prophylaktischen oder kurativen Behandlung von Erkrankungen und Zuständen der vorgenannten Art, wobei im Rahmen des Verfahrens eine pharmazeutisch bzw. pharmakologisch wirksame Menge der zuvor beschriebenen Zusammensetzung nach der vorliegenden Erfindung verabreicht wird, vorzugsweise peroral, insbesondere in Form einer wäßrigen Suspension oder Lösung.

Weitere Ausgestaltungen, Abwandlungen und Variationen der vorliegenden Erfindung sind für den Fachmann beim Lesen der Beschreibung ohne weiteres erkennbar und realisierbar, ohne daß er dabei den Rahmen der vorliegenden Erfindung verläßt.

Die vorliegende Erfindung wird anhand der nachfolgenden Ausführungsbeispiele veranschaulicht, welche die vorliegende Erfindung jedoch keinesfalls beschränken.

### Ausführungsbeispiele

### Herstellung von erfindungsgemäßen pharmazeutischen Zusammensetzungen zur Verwendung als Laxantien

Zur Herstellung einer erfindungsgemäßen Zusammensetzung wurden die folgenden Einwaagen gewählt:

| | |
|---|---|
| Sennesfrüchtetrockenextrakt (10 %) | 506,67 g |
| Plantagosamen (maximale Teilchengröße: 140 µm) | 7.000 g |
| Farbstoff | 76 g |
| Aromastoff | 400 g |
| Süßstoff | 28 g |
| Granulatbildner (Maltodextrin) | 836 g |
| Säuerungsmittel (Citronensäure, wasserfrei) | 1.153 g |
| Wasser (Granulierflüssigkeit) | 4 kg |

Aus den Plantagosamen, dem Sennesfrüchtetrockenextrakt und dem Aromastoff wurde eine Pulvermischung hergestellt. Die übrigen Rohstoffe wurden in Wasser gelöst; die Lösung war klar und wurde nachfolgend als Granulierflüssigkeit verwendet.

Nachfolgend wurde eine Wirbelschichtgranulierung durchgeführt. Zu diesem Zweck wurde die Pulvermischung in die Wirbelschichtgranulierung überführt. Es wurde mit 200 bis 300 m³/h fluidisiert. Nach Erreichen von 70 °C in der Zulufttemperatur wurde mit dem Einsprühen begonnen. Die Zulufttemperatur erhöhte sich während des Prozesses auf bis zu 85 °C. Die Produkttemperatur betrug zwischenzeitlich bis zu 60 °C, im Mittel 40 bis 50 °C. Die Granulierung erfolgte mit einer mittleren eingestellten Sprührate von 46 ml/min, was einer tatsächlichen Sprührate von 29,7 g Granulierflüssigkeit/Minute entsprach. Die Dauer betrug netto 145 Minuten. Das entstandene Granulat wurde ca. 1 Stunde getrocknet, bis der Trocknungsverlust unter 5 % lag. Die maximale Produkttemperatur betrug 60 °C. Die Ausbeute betrug 8 kg.

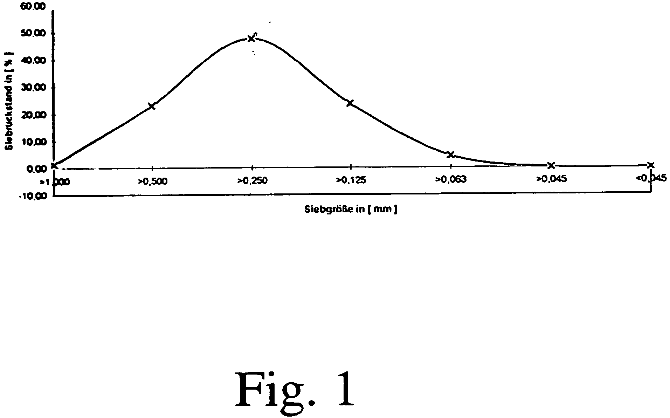

Die Teilchengrößenverteilung des erhaltenen Granulats ist in Fig. 1 wiedergegeben.

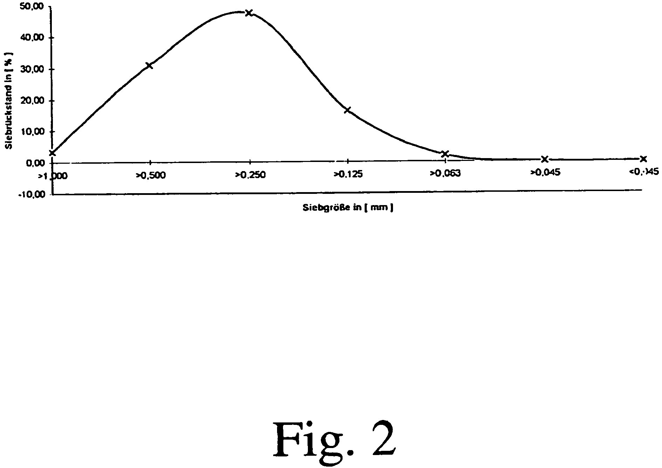

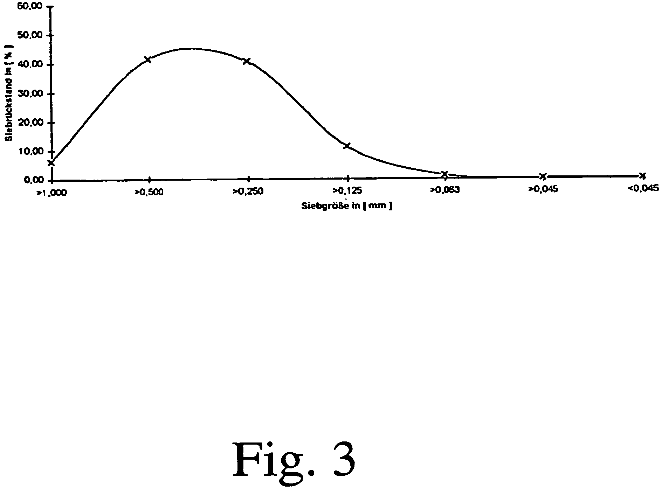

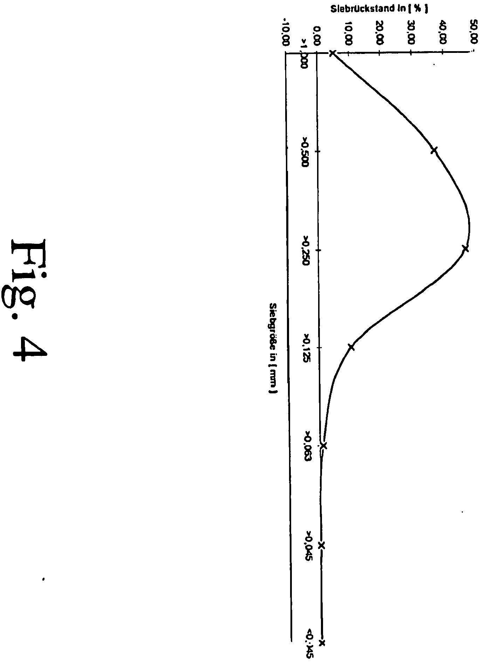

Die zuvor beschriebene Herstellungsweise wurde für die Herstellung von drei weiteren erfindungsgemäßen Zusammensetzungen unter Variation der Granulierbedingungen wiederholt. Die entsprechenden Teilchengrößenverteilungen der erhaltenen Granulate sind in Fig. 2 bis 4 wiedergegeben.

Auf entsprechende Weise wurden auch erfindungsgemäße Zusammensetzungen mit anderen Geschmacksrichtungen hergestellt (z. B. Apfel und Kirsche).

Der erfindungsgemäß eingesetzte Sennesfrüchtetrockenextrakt wurde wie folgt hergestellt: Die geeigneten zerkleinerten Sennesfrüchte wurden in Perkolatoren gegeben; die Perkolatoren wurden mit kaltem Wasser gefüllt, in Reihe geschaltet und aufsteigend perkoliert. Das Perkolat wurde nachfolgend im Dünnextraktbehälter gesammelt und anschließend in der Kurzzeiterhitzung erhitzt. Die ausgefallenen Feststoffe wurden im Separator abgetrennt und der Dünnextrakt mit Hilfe eines Vakuumfallstromverdampfers eingeengt, wobei der eingeengte Extrakt nachfolgend in einer Uperisationsanlage uperisiert wurde. Für die Qualitätskontrolle wurden zur Bestimmung des Trockensubstanzgehaltes Muster bzw. Proben entnommen. Nachfolgend erfolgte eine Sprühtrocknung in einem Sprühturm, wobei die Sprühlösung mit Kohlendioxid versetzt wurde. Abschließend wurde das Sprühprodukt in einem Konusschneckenmischer gemischt, die Mischung gewogen und zwischenverpackt bis zum Einsatz in dem erfindungsgemäßen Herstellungsverfahren. Das Litergewicht des auf diese Weise hergestellten Sennesfrüchtetrockenextraktes lag im Bereich von 100 bis 300 g/l und die Schüttdichte im Bereich von 200 bis 400 g/l. Als Sennesfrüchteausgangsmischung wurde eine 50 : 50-Mischung von Alexandriner-Sennesfrüchten und Tinnevelly-Sennesfrüchten eingesetzt. Die erhaltene Sennesfrüchtetrockenextrakt wies einen Gehalt an Hydroxyanthracenderivaten, berechnet als Sennosid B und bezogen auf den Sennesfrüchtetrockenextrakt, von 10 % auf.

### Herstellung von wäßrigen Suspensionen für die perorale Applikation

Eine wie zuvor hergestellte erfindungsgemäße Zusammensetzung und zwei Vergleichsprodukte des Standes der Technik werden in eine wäßrige Suspension überführt. Zu diesem Zweck werden jeweils 5 g der Zusammensetzungen in je etwa 200 ml Leitungswasser eingerührt.

Die erste Vergleichszusammensetzung ist ein Handelsprodukt auf Basis von indischen Flohsamenschalen (Psyllium), welches daneben weitere Inhaltsstoffe (unter anderem natürliche und künstliche Farb-, Geschmacks-, Aroma- und Süßungsstoffe, Citronensäure, Eisenoxid und Calcium) enthält, aber frei von Sennosiden ist. Die zweite Vergleichszusammensetzung ist eine pulverförmige Zusammensetzung in Form eines feinteiligen Pulvers (mehr als 70 Gew.-% der Teilchen mit mittleren Durchmessern im Bereich von 125 bis 250 µm), welches 52 Gew.-% Plantagosamen, 2,2 Gew.-% Plantagosamenschalen, 12,3 Gew.-% Tinnevelly-Sennesfrüchte und Alexandriner-Sennesfrüchte (Mischungsverhältnis = 1 : 1) entsprechend etwa 0,3 Gew.-% Sennosiden, 8,3 Gew.-% Guar-Gummi, 0,07 Gew.-% feinteilige Kieselsäure, 0,03 Gew.-% Maltodextrin und 25,1 Gew.-% weitere Inhaltsstoffe (d. h. natürliche Aroma-, Farb-, Geschmacks- und Süßungsstoffe) enthält.

Nach Suspension der drei Zusammensetzungen in Wasser bleiben nur die erfindungsgemäße Zusammensetzung und die zweite Vergleichszusammensetzung, welche der DE 103 46 083 A1 der Anmelderin selbst entspricht, über mehr als eine Viertelstunde stabil, während die Vergleichssuspension mit der ersten Vergleichszusammensetzung schon nach wenigen Minuten eine Phasentrennung zeigt und nach einer Viertelstunde geliert ist und einen dicken, untrinkbaren Klumpen auf der Oberfläche bildet; zudem fällt die erste Vergleichszusammensetzung auch nachteilig durch ihren faden Geschmack und das künstliche Aussehen auf.

Nach weiteren drei Stunden zeigte aber auch die wäßrige Suspension mit der zweiten Vergleichszusammensetzung gemäß der DE 103 46 083 A1 eine Phasentrennung, wohingegen die wäßrige Suspension mit der erfindungsgemäßen Zusammensetzung nach wie vor keine Phasentrennung zeigte und auch darüber hinaus noch weitere Stunden phasenstabil und trinkbar blieb.

Im übrigen unterscheiden sich die vorgenannten drei Zusammensetzungen signifikant auch in ihrer laxativen Wirkung: Während die erste Vergleichszusammensetzung auf Basis von indischen Flohsamenschalen (Psyllium) nur eine mäßig abführende Wirkung zeigt, die in schwereren Fällen von Obstipationen nicht immer ausreichend ist, zeigt die zweite Vergleichszusammensetzung eine deutlich besser abführende Wirkung aufgrund der Kombination von physikalischer Wirkung der Plantagosamen/Plantagosamenschalen infolge von Quellung einerseits und pharmakologisch anregender Wirkung der Sennoside andererseits, wobei jedoch aufgrund der Pulverförmigkeit der Mischung keine exakte Teelöffeldosierung möglich ist und andererseits die Gefahr von Risiken (z. B. Klumpenbildung) bei unsachgemäßer Applikation nicht auszuschließen ist. Lediglich bei der erfindungsgemäßen Zusammensetzung kann aufgrund des vorliegenden Granulats eine teelöffelgemäße Dosierung erfolgen und eine vorzeitige Verklumpung auch bei unsachgemäßer Anwendung zumindest im wesentlichen ausgeschlossen werden; zudem ist infolge der Verwendung des Sennesfrüchtetrockenextraktes die Konzentration an Sennosiden derart erhöht, daß zur Erzielung einer vergleichbaren laxativen Wirkung eine deutlich geringere Einnahmemenge (nämlich in etwa die Hälfte) erforderlich ist, was weiterhin die Gefahr auch bei unsachgemäßer Anwendung minimiert.

Im Ergebnis ist es im Rahmen der vorliegenden Erfindung gelungen, eine leistungsstarke pharmazeutische Zusammensetzung zur Verwendung als Laxativum bereitzustellen, welche die eingangs geschilderten Nachteile des Standes der Technik in effizienter Weise vermeidet.

## Patentansprüche

1. Pharmazeutische Zusammensetzung, insbesondere zur Verwendung als Laxans (Abführmittel),
wobei die Zusammensetzung als Granulat vorliegt,
wobei die Zusammensetzung in Kombination und in jeweils pharmazeutisch wirksamen Mengen
(A) 1 bis 15 Gew.-% Sennesfrüchtetrockenextrakt, bezogen auf die Zusammensetzung, wobei der Sennesfrüchtetrockenextrakt ein Droge/Extrakt-Verhältnis von mindestens 2 : 1 aufweist,
(B) 20 bis 90 Gew.-% Plantagosamen *(Plantaginis ovatae Semen),* bezogen auf die Zusammensetzung, wobei auf die Inkorporierung von Plantagosamenschalen verzichtet wird und ausschließlich Plantagosamen eingesetzt wird, und
(C) 3 bis 30 Gew.-% mindestens eines Granulatbildners, bezogen auf die Zusammensetzung,
enthält und
wobei die Zusammensetzung eine definierte Teilchengröße (Korngröße) und/oder Teilchengrößenverteilung (Korngrößenverteilung) aufweist derart, daß mehr als 99 Gew.-% der Teilchen der Zusammensetzung kleiner als 2.000 µm und mehr als 94 Gew.-% der Teilchen kleiner als 1.000 µm sind,
wobei alle vorgenannten Gewichtsangaben jeweils auf das Trockengewicht der Gesamtzusammensetzung bezogen sind.

2. Zusammensetzung nach Anspruch 1,
wobei der Sennesfrüchtetrockenextrakt einen Gehalt an Hydroxyanthracenderivaten, berechnet als Sennosid B und bezogen auf den Sennesfrüchtetrockenextrakt, von mindestens 3 Gew.-%, insbesondere mindestens 5 Gew.-%, vorzugsweise mindestens 7 Gew.-%, besonders bevorzugt mindestens 8 Gew.-%, enthält und/oder
wobei der Sennesfrüchtetrockenextrakt einen Gehalt an Hydroxyanthracenderivaten, berechnet als Sennosid B und bezogen auf den Sennesfrüchtetrockenextrakt, im Bereich von 3 bis 30 Gew.-%, insbesondere 5 bis 25 Gew.-%, vorzugsweise 7 bis 20 Gew.-%, besonders bevorzugt 8 bis 15 Gew.-%, ganz besonders bevorzugt 8 bis 12 Gew.-%, enthält.

3. Zusammensetzung nach einem oder mehreren der vorangehenden Ansprüche, wobei der Sennesfrüchtetrockenextrakt ein Droge/Extrakt-Verhältnis im Bereich von 2 : 1 bis 10 : 1, insbesondere 2,5 : 1 bis 8 : 1, vorzugsweise 3: 1 bis 6 : 1 aufweist.

4. Zusammensetzung nach einem oder mehreren der vorangehenden Ansprüche,
wobei der Sennesfrüchtetrockenextrakt auf Basis von Früchten der Alexandriner-Sennapflanze und/oder der Tinnevelly-Sennapflanze ausgebildet ist und/oder wobei der Sennesfrüchtetrockenextrakt durch Extraktion von Früchten der Alexandriner-Sennapflanze und/oder der Tinnevelly-Sennapflanze erhältlich ist und/oder wobei der Sennesfrüchtetrockenextrakt ein Gemisch verschiedener anthranoider Verbindungen auf Basis von Hydroxyanthracenderivaten, insbesondere Sennosiden, enthält.

5. Zusammensetzung nach Anspruch 4,
wobei die Hydroxyanthracenderivate, insbesondere Sennoside, ausgewählt sind aus der Gruppe der folgenden Verbindungen der allgemeinen Formel (I): 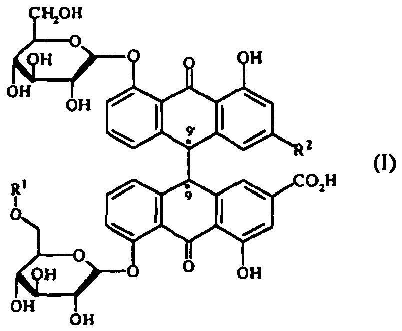 wobei in der allgemeinen Formel (I)
• der Rest R¹ Wasserstoff oder eine Gruppe -CO-CO₂H darstellt,
• der Rest R² eine Gruppe -CO₂H oder -CH₂OH darstellt, jedoch mit der Maßgabe, daß, wenn R¹ eine Gruppe -CO-CO₂H bezeichnet, R² eine Gruppe -CO₂H darstellt,
• die mit dem Zeichen "*" gekennzeichneten Kohlenstoffatome in 9- und 9'-Position des Anthrongerüstes Chiralitätszentren darstellen,
sowie deren Mischungen und/oder Stereoisomeren, insbesondere Enantiomeren und/oder Diastereoisomeren, und/oder Derivaten der vorgenannten Verbindungen und/oder
wobei die Hydroxyanthracenderivate, insbesondere Sennoside, ausgewählt sind aus der Gruppe der folgenden Verbindungen der allgemeinen Formel (I) und deren Mischungen:
| Verbindung | R¹ | R² | 9-9' |
|---|---|---|---|
| (I A) | -H | -CO₂H | R*,R* (threo) |
| (I B) | -H | -CO₂H | R*,S* (erythro) |
| (I C) | -H | -CH₂OH | R*,R* (threo) |
| (I D) | -H | -CH₂OH | R*,S* (erythro) |
| (I E) | -CO-CO₂H | -CO₂H | R*,R* (threo) |
| (I F) | -CO-CO₂H | -CO₂H | R*,S* (erythro) |

6. Zusammensetzung nach einem oder mehreren der vorangehenden Ansprüche, **gekennzeichnet durch** einen Gehalt an Sennesfrüchtetrockenextrakt im Bereich von 2 bis 10 Gew.-%, vorzugsweise 3 bis 8 Gew.-%, besonders bevorzugt 4 bis 7 Gew.-%, ganz besonders bevorzugt 4,5 bis 6,5 Gew.-%, bezogen auf die Zusammensetzung.

7. Zusammensetzung nach einem oder mehreren der vorangehenden Ansprüche, **gekennzeichnet durch** einen Gesamtgehalt an Hydroxyanthracenderivaten, insbesondere Sennosiden, von mindestens 0,1 Gew.-%, insbesondere mindestens 0,2 Gew.-%, vorzugsweise mindestens 0,3 Gew.-%, besonders bevorzugt mindestens 0,4 Gew.-%, ganz besonders bevorzugt mindestens 0,5 Gew.-%, bezogen auf die Zusammensetzung, und/oder **gekennzeichnet durch** einen Gesamtgehalt an Hydroxyanthracenderivaten, insbesondere Sennosiden, im Bereich von 0,1 bis 2 Gew.-%, insbesondere 0,2 bis 1 Gew.-%, vorzugsweise 0,3 bis 0,8 Gew.-%, besonders bevorzugt 0,4 bis 0,7 Gew.-%, bezogen auf die Zusammensetzung.

8. Zusammensetzung nach einem oder mehreren der vorangehenden Ansprüche, **gekennzeichnet durch** einen Gehalt an Plantagosamen im Bereich von 30 bis 90 Gew.-%, vorzugsweise 40 bis 85 Gew.-%, besonders bevorzugt 50 bis 80 Gew.-%, ganz besonders bevorzugt 65 bis 75 Gew.-%, bezogen auf die Zusammensetzung.

9. Zusammensetzung nach einem oder mehreren der vorangehenden Ansprüche,
wobei die Zusammensetzung den oder die Granulatbildner in Mengen von 4 bis 25 Gew.-%, vorzugsweise 5 bis 20 Gew.-%, besonders bevorzugt 6 bis 15 Gew.-%, ganz besonders bevorzugt 7 bis 10 Gew.-%, bezogen auf die Zusammensetzung, enthält und/oder
wobei der Granulatbildner ausgewählt ist aus der Gruppe von Stärkederivaten, Cellulose und Cellulosederivaten, Poly(meth)acrylsäuren und Poly(meth)acrylaten, Gelatine, Polyvinylpyrrolidon, Polyalkylenglykolen, Dextrose (D-Glucose), Lactose, Maltose und Zuckeraustauschstoffen sowie deren Mischungen und/oder
wobei das Granulat durch Wirbelschichtgranulierung erhältlich ist.

10. Zusammensetzung nach Anspruch 9, wobei die Stärkederivate ausgewählt sind aus Stärkeabbauprodukten, insbesondere Dextrinen und Maltodextrinen, vorzugsweise Maltodextrinen.

11. Zusammensetzung nach einem der vorangehenden Ansprüche, wobei die Zusammensetzung außerdem mindestens ein weiteres Additiv, Inhaltsstoff und/oder Zusatzstoff enthält, insbesondere ausgewählt aus der Gruppe von Farbstoffen wie natürlichen oder naturidentischen Farbstoffen, Geschmacksmitteln, Geschmacksverstärkern und Aromastoffen, Süßungsmitteln, Säuerungsmitteln, Konservierungsmitteln, Stabilisatoren und Costabilisatoren, Elektrolyten, Mineralien und Mineralstoffen, Vitaminen, Füllstoffen, Fließmitteln und Verarbeitungshilfsmitteln sowie Mischungen der vorgenannten Verbindungen.

12. Zusammensetzung nach einem oder mehreren der vorangehenden Ansprüche,
wobei die Zusammensetzung, insbesondere das Granulat, eine mittlere Teilchengröße (mittlere Korngröße) im Bereich von 50 bis 400 µm, insbesondere 50 bis 300 µm, vorzugsweise 75 bis 275 µm, aufweist und/oder
wobei der Hauptmassenanteil der Zusammensetzung, insbesondere des Granulats, vorzugsweise mehr als 55 Gew.-%, insbesondere mehr als 60 Gew.-%, bevorzugt mehr als 65 Gew.-%, besonders bevorzugt mehr als 70 Gew.-%, ganz besonders bevorzugt mehr als 75 Gew.-% der Teilchen der Zusammensetzung, Korngrößen im Bereich von 50 bis 1.000 µm, bevorzugt mit einem Maximum der Verteilung insbesondere bei Teilchengrößen im Bereich von 65 bis 500 µm, vorzugsweise 125 bis 450 µm, aufweisen.

13. Zusammensetzung nach einem oder mehreren der vorangehenden Ansprüche, wobei die Zusammensetzung, insbesondere das Granulat, folgende Teilchengrößenverteilung (Korngrößenverteilung) aufweist:
• Teilchengrößen > 2.000 µm:
weniger als 1 Gew.-%, insbesondere 0 Gew.-%;
• 2.000 µm ≤ Teilchengrößen > 1.000 µm:
0,5 bis 10 Gew.-%, insbesondere 1 bis 10 Gew.-%, vorzugsweise 1 bis 6 Gew.-%;
• 1.000 µm ≤ Teilchengrößen > 500 µm:
15 bis 45 Gew.-%, insbesondere 20 bis 40 Gew.-%, vorzugsweise 25 bis 40 Gew.-%;
• 500 µm ≤ Teilchengrößen > 250 µm:
35 bis 60 Gew.-%, insbesondere 40 bis 55 Gew.-%, vorzugsweise 40 bis 50 Gew.-%;
• 250 µm ≤ Teilchengrößen > 125 µm:
5 bis 30 Gew.-%, insbesondere 10 bis 25 Gew.-%, vorzugsweise 10 bis 20 Gew.-%;
• 125 µm ≤ Teilchengrößen > 63 µm:
0,5 bis 5 Gew.-%, insbesondere 0,5 bis 4 Gew.-%, vorzugsweise 1 bis 3 Gew.-%;
• 63 µm ≤ Teilchengrößen > 45 µm:
0 bis 3 Gew.-%, insbesondere 0 bis 1 Gew.-%, vorzugsweise 0 Gew.-%;
wobei alle vorgenannten Gewichtsangaben jeweils auf das Trockengewicht der Gesamtzusammensetzung bezogen sind und mit der Maßgabe, daß die Summe der Gewichtsprozentangaben 100 Gew.-% ergibt.

14. Verfahren zur Herstellung einer Zusammensetzung nach den Ansprüchen 1 bis 13, wobei die Wirk- und/oder Inhaltsstoffe der Zusammensetzung, wie zuvor definiert, insbesondere (A) der Sennesfrüchtetrockenextrakt und (B) der Plantagosamen (*Plantaginis ovatae Semen)* sowie gegebenenfalls weitere Bestandteile der Zusammensetzung, gegebenenfalls nach vorangehender Einstellung der Teilchengrößen, insbesondere mittels Zerkleinerung, einer Granulierung, vorzugsweise im Wirbelschichtverfahren, in Gegenwart (C) mindestens eines Granulatbildners unterzogen werden.

15. Zusammensetzung nach den Ansprüchen 1 bis 13 zur Verwendung als Laxativum (Abführmittel).

## Claims

1. A pharmaceutical composition, in particular for use as a laxative (purgative), wherein the composition is present as a granulate,
wherein the composition contains, in combination and in pharmaceutically effective amounts, respectively
(A) 1 to 15% by weight senna fruit dry extract, based on the composition, wherein the senna fruit dry extract has a drug/extract ratio of at least 2:1,
(B) 20 to 90% by weight alpine plantain seed (Plantaginis ovatae semen), based on the composition, wherein the incorporation of alpine plantain seed shells is omitted, and alpine plantain seed is utilized exclusively, and
(C) 3 to 30% by weight of at least one granulator, based on the composition,
and
wherein the composition has a defined particle size (grain size) and/or particle size distribution (grain size distribution) such that more than 99% by weight of the particles of the composition are smaller than 2,000 µm, and more than 94% by weight of the particles are smaller than 1,000 µm,
wherein all weights stated above are each based on the dry weight of the total composition.

2. The composition according to claim 1,
wherein the senna fruit dry extract comprises a content of hydroxy anthracene derivatives, calculated as sennsoid B, and based on the senna fruit dry extract, of at least 3% by weight, in particular at least 5% by weight, preferably at least 7% by weight, particularly preferred at least 8% by weight, and/or
wherein the senna fruit dry extract comprises a content of hydroxy anthracene derivatives, calculated as sennsoid B, and based on the senna fruit dry extract, in the range of 3 to 30% by weight, in particular 5 to 25% by weight, preferably 7 to 20% by weight, particularly preferred 8 to 15% by weight, most preferred 8 to 12% by weight.

3. The composition according to one or more of the previous claims, wherein the senna fruit dry extract comprises a drug/extract ratio in the range of 2:1 to 10:1, in particular 2.5:1 to 8:1, preferably 3:1 to 6:1.

4. The composition according to one or more of the previous claims,
wherein the senna fruit dry extract is embodied based on the fruit of the Alexandrine senna plant, and/or the Tinnevelly senna plant, and/or wherein the senna fruit dry extract is obtainable by means of extraction from the fruit of the Alexandrine senna plant, and/or the Tinnevelly senna plant, and/or
wherein the senna fruit dry extract contains a mixture of different anthranoid compounds based on hydroxy anthracene derivatives, in particular sennosides.

5. The composition according to claim 4,
wherein the hydroxy anthracene derivatives, in particular sennoside, are selected from the group of the following compounds of the general formula (I): 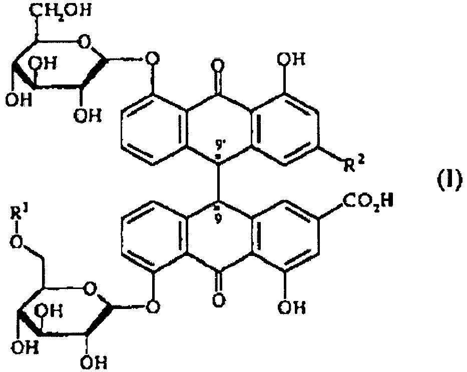 wherein in the general formula (I)
• the residue R¹ represents hydrogen, or a group -CO-CO₂H,
• the residue R² represents a group -CO₂H or -CH₂OH, however, provided that, if R¹ denotes a group -CO-CO₂H, then R² represents a group -CO₂H,
• the carbon atoms in the 9 and 9' position of the anthrone framework, denoted by the symbol "*", represent chirality centers,
as well as the mixtures and/or stereoisomers, in particular enantiomers and/or diastereoisomers thereof, and/or the derivatives of the compounds mentioned above, and/or
wherein the hydroxy anthracene derivatives, in particular sennosides, are selected from the group of the following compounds of the general formula (I) and the mixtures thereof:
| Compound | R¹ | R² | 9-9' |
|---|---|---|---|
| (I A) | -H | -CO₂H | R*,R* (threo) |
| (I B) | -H | -CO₂H | R*,S* (erythro) |
| (I C) | -H | -CH₂OH | R*,R* (threo) |
| (I D) | -H | -CH₂OH | R*,S* (erythro) |
| (I E) | -CO-CO₂H | -CO₂H | R*,R* (threo) |
| (I F) | -CO-CO₂H | -CO₂H | R*,S* (erythro) |

6. The composition according to one or more of the previous claims, **characterized by** a content of senna fruit dry extract in the range of 2 to 10% by weight, preferably 3 to 8% by weight, particularly preferred 4 to 7% by weight, most preferred 4.5 to 6.5% by weight, based on the composition.

7. The composition according to one or more of the previous claims, **characterized by** a total content of hydroxy anthracene derivatives, in particular sennosides, of at least 0.1% by weight, in particular at least 0.2% by weight, preferably at least 0.3% by weight, particularly preferred at least 0.4% by weight, most preferred at least 0.5% by weight, based on the composition, and/or **characterized by** a total content of hydroxy anthracene derivatives, in particular sennosides, in the range of 0.1 to 2% by weight, in particular 0.2 to 1% by weight, preferably 0.3 to 0.8% by weight, particularly preferred 0.4 to 0.7% by weight, based on the composition.

8. The composition according to one or more of the previous claims, **characterized by** a total content of alpine plantain seed in the range of 30 to 90% by weight, preferably 40 to 85% by weight, particularly preferred 50 to 80% by weight, most preferred 65 to 75% by weight, based on the composition.

9. The composition according to one or more of the previous claims,
wherein the composition comprises the granulator(s) in amounts from 4 to 25% by weight, preferably 5 to 20% by weight, particularly preferred 6 to 15% by weight, most preferred 7 to 10% by weight, based on the composition, and/or
wherein the granulator is selected from the group of starch derivatives, cellulose, and cellulose derivatives, poly(meth)acrylic acids, and poly(meth)acrylates, gelatin, polyvinyl pyrrolidone, polyalkylene glycols, dextrose (D-glucose), lactose, maltose, and sugar substitutes, as well as the mixtures thereof, and/or
wherein the granulate may be obtained by means of fluidized bed granulation.

10. The composition according to claim 9, wherein the starch derivatives are selected from starch metabolites, in particular dextrins and maltodextrins, preferably maltodextrins.

11. The composition according to one of the previous claims, wherein the composition further comprises at least one additional additive, active ingredient, and/or adjuvant, in particular selected from the group of dyes, such as natural or nature identical dyes, flavorings, flavor enhancers, and aromatics, sweeteners, acidifiers, preservatives, stabilizers, and costabilizers, electrolytes, minerals, and mineral compounds, vitamins, fillers, eluents, and processing aids, as well as the mixtures of the compounds mentioned above.

12. The composition according to one of the previous claims,
wherein the composition, in particular the granulate, has a mean particle size (mean grain size) in the range of 50 to 400 µm, in particular 50 to 300 µm, preferably 75 to 275 µm, and/or
wherein the bulk portion of the composition, in particular of the granulate, preferably comprises more than 55% by weight, in particular more than 60% by weight, preferably more than 65% by weight, particularly preferred more than 70% by weight, most preferred more than 75% by weight of the particles of the composition, with grain sizes in the range of 50 to 1,000 µm, preferably at a maximum of the distribution, in particular at particle sizes in the range of 65 to 500 µm, preferably 125 to 450 µm.

13. The composition according to one of the previous claims, wherein the composition, in particular, the granulate, has the following particle size distribution (grain size distribution):
• particle sizes > 2,000 µm:
Less than 1% by weight, in particular 0% by weight;
• 2,000 µm ≤ particle sizes > 1,000 µm:
0.5 to 10% by weight, in particular 1 to 10% by weight, preferably 1 to 6% by weight;
• 1,000 µm ≤ particle sizes > 500 µm:
15 to 45% by weight, in particular 20 to 40% by weight, preferably 25 to 40% by weight;
• 500 µm ≤ particle sizes > 250 µm:
35 to 60% by weight, in particular 40 to 55% by weight, preferably 40 to 50% by weight;
• 250 µm ≤ particle sizes > 125 µm:
5 to 30% by weight, in particular 10 to 25% by weight, preferably 10 to 20% by weight;
• 125 µm ≤ particle sizes > 63 µm:
0.5 to 5% by weight, in particular 0.5 to 4% by weight, preferably 1 to 3% by weight;
• 63 µm ≤ particle sizes > 45 µm:
0 to 3% by weight, in particular 0 to 1% by weight, preferably 0% by weight;
wherein all weights stated above are each based on the dry weight of the total composition, and provided that the sum of the weight percentages results in 100% by weight.

14. A method for the production of a composition according to claims 1 to 13, wherein the active agents and/or ingredients of the composition, as defined above, in particular (A) of the senna fruit dry extract, and (B) of the alpine plantain seed (Plantaginis ovatae semen), as well as optionally any additional components of the composition, are subjected to granulation, preferably by means of the fluidized bed method, in the presence (C) of at least one granulator, optionally after the previous adjustment of the particle sizes, in particular by means of grinding.

15. The composition according to claims 1 to 13 for use as a laxative (purgative).

## Revendications

1. Composition pharmaceutique, en particulier pour utilisation en tant que laxatif (purgatif),
la préparation se présentant sous forme d'un granulé,
la préparation contenant, en combinaison et dans chaque cas en des quantités pharmaceutiquement actives
(A) 1 à 15 % en poids d'extrait sec de séné, par rapport à la composition, l'extrait sec de séné présentant un rapport drogue/extrait d'au moins 2:1,
(B) 20 à 90 % en poids de graines de psyllium (*Plantaginis ovatae Semen*), par rapport à la composition, en renonçant à l'incorporation de coques de graines de psyllium, et en utilisant exclusivement des graines de psyllium, et
(C) 3 à 30 % en poids d'au moins un agent de granulation, par rapport à la composition, et
la composition présentant une grosseur de particules (granulométrie) ou une répartition des grosseurs de particules (répartition granulométrique) parfaitement définie, de telle sorte que plus de 99 % en poids des particules de la composition soient inférieures à 2000 µm, et que plus de 94 % en poids des particules soient plus petites que 1000 µm,
toutes les indications de poids ci-dessus étant chacune rapportées au poids sec de la composition totale.

2. Composition selon la revendication 1,
l'extrait sec de séné ayant une teneur en dérivés d'hydroxyanthracènes, calculé en sennoside B et par rapport à l'extrait sec de séné, d'au moins 3 % en poids, en particulier d'au moins 5 % en poids, de préférence d'au moins 7 % en poids, d'une manière particulièrement préférée d'au moins 8 % en poids, et/ou
l'extrait sec de séné ayant une teneur en dérivés d'hydroxyanthracènes, calculé en sennoside B et par rapport à l'extrait sec de séné, comprise dans la plage de 3 à 30 % en poids, en particulier de 5 à 25 % en poids, de préférence de 7 à 20 % en poids, d'une manière particulièrement préférée de 8 à 15 % en poids, d'une manière tout particulièrement préférée de 8 à 12 % en poids.

3. Composition selon l'une ou plusieurs des revendications précédentes, l'extrait sec de séné présentant un rapport drogue/extrait compris dans la plage de 2:1 à 10:1, en particulier de 2,5:1 à 8:1, de préférence de 3:1 à 6:1.

4. Composition selon l'une ou plusieurs des revendications précédentes,
l'extrait sec de séné étant réalisé à base de fruits de séné d'Alexandrie et/ou de séné de Tinnevelly, et/ou l'extrait sec de séné pouvant être obtenu par extraction de fruits de séné d'Alexandrie et/ou de séné de Tinnevelly, et/ou
l'extrait sec de séné contenant un mélange de différents composés anthranoïdes à base de dérivés d'hydroxyanthracènes, en particulier des sennosides.

5. Composition selon la revendication 4,
dans laquelle les dérivés d'hydroxyanthracènes, en particulier les sennosides, sont choisis dans le groupe des composés suivants de formule générale (I) : 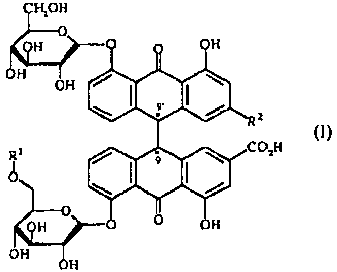 dans laquelle, dans la formule générale (I)
- le radical R¹ représente un atome d'hydrogène ou un groupe -CO-CO₂H,
- le radical R² représente un groupe -CO₂H ou -CH₂OH, mais à la condition que, quand R¹ désigne un groupe -CO-CO₂H, R² représente un groupe -CO₂H,
- les atomes de carbone marqués du signe "*" en positions 9 et 9' du squelette anthrone représentent des centres de chiralité,
ainsi que leurs mélanges et/ou stéréo-isomères, en particulier leurs énantiomères et/ou diastéréo-isomères, et/ou les dérivés des composés mentionnés ci-dessus, et/ou
dans laquelle les dérivés d'hydroxyanthracènes, en particulier les sennosides, sont choisis dans le groupe des composés suivants de formule générale (I) et de leurs mélanges :
| Composé | R¹ | R² | 9-9' |
|---|---|---|---|
| (IA) | -H | -CO₂H | R*,R* (thréo) |
| (IB) | -H | -CO₂H | R*,S* (érythro) |
| (IC) | -H | -CH₂OH | R*,R* (thréo) |
| (ID) | -H | -CH₂OH | R*,S* (érythro) |
| (IE) | -CO-CO₂H | -CO₂H | R*,R* (thréo) |
| (IF) | -CO-CO₂H | -CO₂H | R*,S* (érythro) |

6. Composition selon l'une ou plusieurs des revendications précédentes, **caractérisée par** une teneur en extrait sec de séné comprise dans la plage de 2 à 10 % en poids, de préférence de 3 à 8 % en poids, d'une manière particulièrement préférée de 4 à 7 % en poids, d'une manière tout particulièrement préférée de 4,5 à 6,5 % en poids, par rapport à la composition.

7. Composition selon l'une ou plusieurs des revendications précédentes, **caractérisée par** une teneur totale en dérivés d'hydroxyanthracènes, en particulier des sennosides, d'au moins 0,1 % en poids, en particulier d'au moins 0,2 % en poids, de préférence d'au moins 0,3 % en poids, d'une manière particulièrement préférée d'au moins 0,4 % en poids, d'une manière tout particulièrement préférée d'au moins 0,5 % en poids, par rapport à la composition, et **caractérisée par** une teneur totale en dérivés d'hydroxyanthracènes, en particulier des sennosides, comprise dans la plage de 0,1 à 2 % en poids, en particulier de 0,2 à 1 % en poids, de préférence de 0,3 à 0,8 % en poids, d'une manière particulièrement préférée de 0,4 à 0,7 % en poids, par rapport à la composition.

8. Composition selon l'une ou plusieurs des revendications précédentes, **caractérisée par** une teneur en graines de psyllium comprise dans la plage de 30 à 90 % en poids, de préférence de 40 à 85 % en poids, d'une manière particulièrement préférée de 50 à 80 % en poids, d'une manière tout particulièrement préférée de 65 à 75 % en poids, par rapport à la composition.

9. Composition selon l'une des revendications précédentes, la composition contenant le ou les agents de granulation ont des quantités de 4 à 25 % en poids, de préférence de 5 à 20 % en poids, d'une manière particulièrement préférée de 6 à 15 % en poids, d'une manière tout particulièrement préférée de 7 à 10 % en poids, par rapport à la composition, et/ou
l'agent de granulation étant choisi dans le groupe des dérivés de l'amidon, de la cellulose et des dérivés de la cellulose, des acides poly(méth)acryliques et des poly(méth)acrylates, de la gélatine, de la polyvinylpyrrolidone, des polyalkylèneglycols, du dextrose (D-glucose), du lactose, du maltose et des succédanés du sucre, ainsi que de leurs mélanges, et/ou
le granulé pouvant être obtenu par granulation en lit fluidisé.

10. Composition selon la revendication 9, dans laquelle le dérivé de l'amidon est choisi parmi les produits de dégradation de l'amidon, en particulier les dextrines et les maltodextrines, de préférence les maltodextrines.

11. Composition selon l'une des revendications précédentes, la composition contenant en outre au moins un additif, un ingrédient et/ou un produit d'addition supplémentaire, en particulier choisi dans le groupe des colorants tels que les colorants naturels ou identiques au naturel, les agents de sapidité, les exhausteurs de goût et les substances aromatisantes, les édulcorants, les acidifiants, les conservateurs, les stabilisants et les co-stabilisants, les électrolytes, les minéraux et les substances minérales, les vitamines, les matières de charge, les agents de fluidité et les auxiliaires de mise en oeuvre, ainsi que les mélanges des composés mentionnés ci-dessus.

12. Composition selon l'une ou plusieurs des revendications précédentes,
la composition, en particulier le granulé, présentant une grosseur moyenne des particules (granulométrie moyenne) comprise dans la plage de 50 à 400 µm, en particulier de 50 à 300 µm, de préférence de 75 à 275 µm, et/ou
dans laquelle la proportion pondérale principale de la composition, en particulier du granulé, de préférence plus de 55 % en poids, en particulier plus de 60 % en poids, de préférence plus de 65 % en poids, d'une manière particulièrement préférée plus de 70 % en poids, d'une manière tout particulièrement préférée plus de 75 % en poids des particules de la composition, présentent une grosseur de grain comprise dans la plage de 50 à 1000 µm, de préférence avec un maximum de la distribution en particulier pour des granulométries comprises dans la plage de 65 à 500 µm, de préférence de 125 à 450 µm.

13. Composition selon l'une ou plusieurs des revendications précédentes, la composition, en particulier le granulé, ayant la distribution des grosseurs de particules (distribution granulométrique) suivante :
- granulométrie > 2000 µm : moins de 1 % en poids, en particulier 0 % en poids ;
- 2000 µm ≤ granulométrie > 1000 µm : 0,5 à 10 % en poids, en particulier 1 à 10 % en poids, de préférence 1 à 6 % en poids ;
- 1000 µm ≤ granulométrie > 500 µm : 15 à 45 % en poids, en particulier 20 à 40 % en poids, de préférence 25 à 40 % en poids ;
- 500 µm ≤ granulométrie > 250 µm : 35 à 60 % en poids, en particulier 40 à 55 % en poids, de préférence 40 à 50 % en poids ;
- 250 µm ≤ granulométrie > 125 µm : 5 à 30 % en poids, en particulier 10 à 25 % en poids, de préférence 10 à 20 % en poids ;
- 125 µm ≤ granulométrie > 63 µm : 0,5 à 5 % en poids, en particulier 0,5 à 4 % en poids, de préférence 1 à 3 % en poids ;
- 63 µm ≤ granulométrie > 45 µm : 0 à 3 % en poids, en particulier 0 à 1 % en poids, de préférence 0 % en poids ; toutes les indications de poids ci-dessus étant chacune rapportées au poids sec de la composition totale, et à la condition que la somme des indications de pourcentage en poids soit de 100 % en poids.

14. Procédé de fabrication d'une composition selon les revendications 1 à 13, dans lequel on soumet à une granulation, de préférence par le procédé en lit fluidisé, en présence (C) d'au moins un agent de granulation, les principes actifs et/ou ingrédients de la composition, tels que définis ci-dessus, en particulier (A) l'extrait sec de séné et (B) la graine de psyllium *(Plantaginis ovatae Semen),* ainsi qu'éventuellement d'autres constituants de la composition, éventuellement après un ajustement préalable des granulométries, en particulier par broyage.

15. Composition selon les revendications 1 à 13 pour utilisation en tant que laxatif (purgatif).
